(19) Europäisches Patentamt

European Patent Office

Office européen des brevets

(11) **EP 1 347 786 B1**

(12) **EUROPEAN PATENT SPECIFICATION**

(45) Date of publication and mention
of the grant of the patent:
**27.09.2006 Bulletin 2006/39**

(51) Int Cl.:
**A61L 2/20** *(2006.01)* **B65B 55/18** *(2006.01)*

(21) Application number: **01997140.7**

(22) Date of filing: **28.12.2001**

(86) International application number:
**PCT/US2001/050516**

(87) International publication number:
**WO 2002/058746 (01.08.2002 Gazette 2002/31)**

(54) **MEDICAL ARTICLES STERILIZATION METHOD AND DEVICE**

VERFAHREN UND VORRICHTUNG ZUR STERILISATION VON MEDIZINISCHEN
GEGENSTÄNDEN

DISPOSITIF ET PROCEDE DE STERILISATION D'ARTICLES MEDICAUX

(84) Designated Contracting States:
**DE FR GB IT NL**

(30) Priority: **28.12.2000 US 258327 P**
**05.04.2001 US 826420**

(43) Date of publication of application:
**01.10.2003 Bulletin 2003/40**

(60) Divisional application:
**04014882.7 / 1 475 106**

(73) Proprietor: **KIMBERLY-CLARK WORLDWIDE, INC.**
**Neenah, Wisconsin 54957-0349 (US)**

(72) Inventor: **MCGOWAN, James, E., Jr.**
**Marietta, GA 30066 (US)**

(74) Representative: **Bertram, Rainer**
**Grünecker, Kinkeldey,**
**Stockmair & Schwanhäusser**
**Anwaltssozietät**
**Maximilianstrasse 58**
**80538 München (DE)**

(56) References cited:
**FR-A- 2 769 289** **US-A- 3 716 961**
**US-A- 5 472 702** **US-A- 5 749 203**
**US-A- 5 868 244**

**Description**

As is generally known, many disposable and reusable medical articles formed from a fabric require sterilization prior to their use. Numerous sterilization processes are available and include radiation, steam, plasma discharge, and sterilization via sterilizing gas. With regard to sterilization via sterilizing gas, one of the more traditional sterilizing gases is ethylene oxide. Well known sterilization processes utilizing ethylene oxide include a chamber sterilization process.

Traditionally, the chamber sterilization process includes four phases: (i) preconditioning, (ii) sterilization, (iii) degassing, and (iv) quarantining. In the preconditioning phase, the medical articles to be sterilized are already sealed in their final packaging. The medical articles are first palletized and then placed in a preconditioning room. The temperature and the humidity in this preconditioning room are set generally between 37.8°C and 60°C (about 100°F. and about 140°F.) and between about 40 and about 80 percent relative humidity, respectively. These conditions are maintained throughout the preconditioning phase, which may take from about 12 to about 72 hours to complete.

The sterilization phase generally involves transferring the pelletized preconditioned articles from the preconditioning room to a sterilization chamber. The size of the sterilization chamber may range from a few cubic meters to 99.1 m$^3$ (3500 cubic feet) or more. The temperature within a sealed sterilization chamber may range from 37.8°C and 60°C (100°F. to 140°F). Additionally, some of the gases within the sealed sterilization chamber may be evacuated such that the pressure therein may be reduced to about 30 to about 650 millibars. By creating a partial vacuum within the sealed sterilization chamber, dilution of the ethylene oxide and risk of fire by ethylene oxide ignition are reduced.

Once under the partial vacuum, the relative humidity within the sterilization chamber is maintained between about 30 and about 80 percent by the injection of water vapor, generally in the form of low pressure steam at less than 103 KPa (15 psi). Following steam injection, to assure all of the articles within the sealed sterilization chamber are moistened, a period of time, generally referred to as a "dwell period," is permitted to lapse.

Once the dwell period has lapsed, a sterilizing gas, such as for example a mixture of ethylene oxide and nitrogen, is introduced into the sterilization chamber. Following the introduction of the sterilizing gas, the pressure level inside the chamber may range from about 500 millibars to about 2300 millibars. The concentration of ethylene oxide within the chamber is generally at least 400 milligrams per liter (mg/l) and may be as high as 1500 mg/l or higher. The duration of exposure to ethylene oxide may be from about 2-12 hours or longer, depending upon several factors, including temperature, pressure, humidity, the specific sterilant mixture being used, and the products being sterilized.

After the articles have been exposed to the sterilizing gas for a sufficient time, the sterilizing gas is evacuated from the chamber by a series of vacuums and air or nitrogen rinses. When ethylene oxide is used, due to its potential flammability in oxygen or air, the chamber is usually rinsed with an inert gas, such as nitrogen.

The degassing phase follows the sterilization phase. Degassing generally involves moving the sterilized, pelletized products from the sterilization chamber to a degassing or aeration room. The temperature in the degassing room is generally maintained between 32.2°C and 60°C (about 90°F. and about 140°F).

In the last phase, the quarantine phase, the articles exiting the degassing room are warehoused in a quarantine area. Samples are removed and tested for sterility. While awaiting sterility verification, additional degassing of the articles may occur. Quarantining and sterility verification may take from 2 to 14 days. As such, the traditional chamber sterilization process, excluding quarantine time, generally may take from between about 48 and about 72 hours for many medical articles.

While the above described process is effective for sterilizing medical articles, the process has several drawbacks. One such drawback in is the length of time required to sterilize. Another drawback is the concentration of ethylene oxide used during the sterilization phase. At a concentration of ethylene oxide between about 400 mg/l and about 1500 mg/1, toxicity and flammability present significant safety concerns.

In response to these problems, a form, fill and seal process has been proposed as disclosed in US-A-5749203. In the form, fill and seal process, an article to be sterilized is placed in a housing defined in part by a preformed bottom web, sized for supporting the article to be sterilized. Then, a top web is placed over the article and the preformed bottom web. Together these form all sides of the housing within a sterilization-sealing station, a ported nozzle is positioned between the top and bottom webs for selective movement of gases into and out of the housing. Upon the evacuation of at least some of the air from the housing via the ported nozzle, steam is introduced into the housing through the ported nozzle.

After the housing has been sufficiently pressurized with steam, a sterilizing gas is introduced via the ported nozzle. Upon sufficient pressurization of the housing, the contacting portions of the top and bottom webs are sealed together by a sealing process, such as by heat sealing, thus closing the housing.

From the form, fill and seal device, the articles, now encased within the closed housing, are conveyed from the sterilization-sealing station to a heating and degassing area. The articles are kept in this area for at least four hours at an elevated temperature, perhaps in the range of 21°C to 71.1°C (about 70°F. to about 160°F). By storing the articles, residual sterilizing gas within the package or housing is allowed to dissipate. By heating the article, the sterilization process is carried to completion. The heat may also assist in degassing.

After a sufficient time has elapsed, the articles are removed from the heating and degassing area, perhaps by a conveyor system. Once removed from the sealed area, the housings are stored in a quarantine area until tested to verify the sterility of the article and to measure the levels of residual sterilizing gas present. Upon satisfactorily meeting the test and verification, the packaged articles are suitable for distribution.

While the form, fill and seal process has advantages over the chamber sterilization process, there is room for improvement. In the form, fill and seal process, steam is injected onto a cold article through a nozzle. The steam may condense on the article, thereby causing water spots when dried. If the steam condensation is large enough, the package can fill up with water. The nozzle significantly increases the cost of the form, fill and seal equipment.

In the form, fill and seal process, the articles are stored within the degassing area and the quarantine area for rather long periods of time. This extended storage is necessary in part to ensure that the medical articles are completely sterilized. That is, the medical articles may not be completely sterilized within the sterilization-sealing station. Sterilization may not be complete until sometime later, when the articles are located within the quarantine area. Unfortunately, the additional time increases the overall cost of the sterilization process.

SUMMARY OF THE INVENTION

One possible way to achieve more complete sterilization in the form, fill and seal device is to allow the articles to remain in the sterilization-sealing station of the form, fill and seal device for a longer period of time. However, the sterilization-sealing station is expensive. Therefore, to maximize throughput, medical articles should pass through the sterilization-sealing station rapidly. The inventors have found that the rate of sterilization is dependent upon the temperature of the medical articles. To achieve better sterilization, one aspect of the present invention may improve heating of the medical articles while in the sterilization-sealing station of the form, fill and seal device.

In response to the foregoing possible areas of improvement, an improved form, fill and seal method and device are proposed. The medical article sterilization device has a pretreatment area for medical articles, a device to form a housing in a first web, an article loading station, an alignment device and a sterilization-sealing station. The pretreatment area has a heat source to heat the medical articles. At the article loading station a medical article heated in the pretreatment area is loaded into the housing in the first web. The alignment device aligns a second web with the first web. At the sterilization-sealing station the first web and the second web, with the medical article loaded into the housing, are sterilized, and then the first and second webs are sealed together. Gas injection pins may be provided at the sterilization-sealing station to inject gas into the housing, between the first and second webs. The sterilization-sealing station may have a steam source to inject steam into the housing, between the first and second webs.

Alternatively, substantially no moisture may be supplied to the medical articles at the sterilization-sealing station. In this case, the pretreatment area could have a steam source to supply moisture to the medical articles. A vacuum source and a controller may be provided at the sterilization-sealing station. The vacuum source evacuates the housing to remove moisture from the medical articles, and the controller maintains the pressure in the housing so as to allow some moisture to remain with the medical articles.

BRIEF DESCRIPTION OF THE DRAWINGS

The invention will be readily understood by reference to the following description of embodiments described by way of example only, with reference to the accompanying drawings in which like reference characters represent like elements, wherein:

Fig. 1- is a schematic layout showing the overall form, fill and seal device, a degassing room and associated elements;
Fig. 2 is a schematic layout of a pretreatment area through which articles may be sent prior to being packaged and sterilized;
Fig. 3 is a side view of a form, fill and seal device shown in Fig. 1;
Fig. 4 is a top view of a sterilization-sealing station included in the form, fill and seal device shown in Figs. 1 and 3;
Fig. 5A is a cross-sectional view of the sterilization-sealing station shown in Figs. 1, 3 and 4, taken through line V-V of Fig. 4, illustrating the top and bottom webs clamped together, but not sealed;
Fig. 5B is a cross-sectional view of the sterilization-sealing station shown in Figs. 1, 3 and 4, taken through line V-V of Fig. 4, illustrating the top and bottom webs clamped together and sealed;
Fig. 5C is a cross-sectional view of the sterilization-sealing station shown in Figs. 1, 3 and 4, taken through line V-V of Fig. 4, illustrating the top and bottom webs being released from the sterilization-sealing station;
Fig. 6 is a top view of top and bottom webs, between which articles are packaged and sterilized;
Fig. 7A is an enlarged cross-sectional view of a portion of the sterilization-sealing station shown in Figs. 1, 3 and 4 taken through line V-V of Fig. 4, illustrating gas injection;
Fig. 7B is an enlarged cross-sectional view of a portion of the sterilization-sealing station shown in Figs. 1, 3 and 4, taken through line VII BD-VII BD of Fig. 4, illustrating gas injection;
Fig. 7C is an enlarged cross-sectional view of a portion of the sterilization-sealing station shown in Figs. 1, 3 and 4, taken through line V-V of Fig. 4, illustrating a sealing operation; and
Fig. 7D is an enlarged cross-sectional view of a portion of the sterilization-sealing station shown in Figs. 1, 3 and 4, taken through line VII BD-VII BD of Fig. 4, illustrating a sealing operation.

DETAILED DESCRIPTION OF THE EMBODIMENTS

The present invention will now be described with reference to embodiments and examples which are given by way of example only, not limitation.

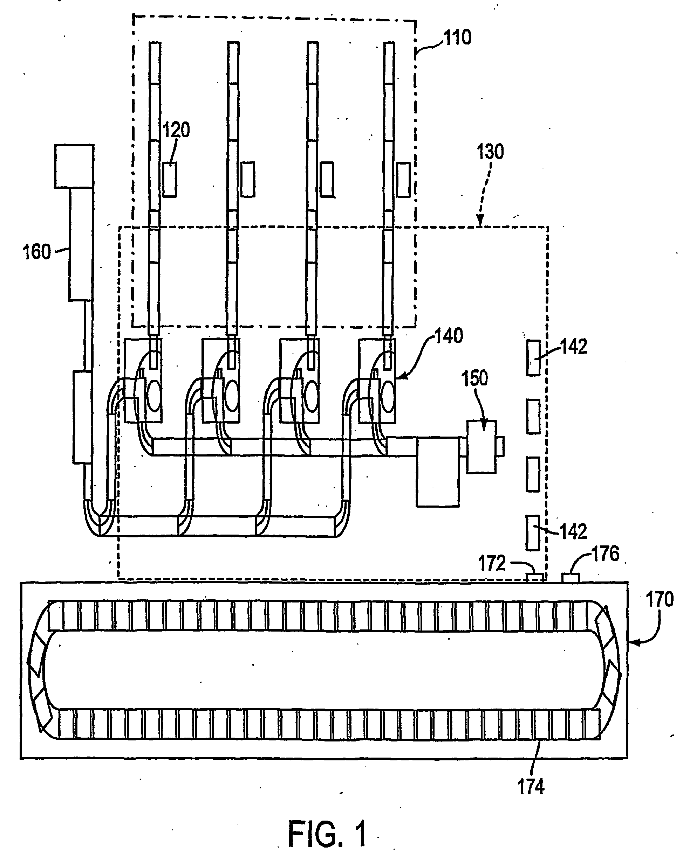

Fig. 1 is a layout showing the overall form, fill and seal device, a degassing room and associated elements. The form, fill and seal device is represented by reference numeral 110 and includes an article loading area 120, at which articles are packaged into a housing. Within an area 130, the articles are sterilized and the packaging is sealed. The area 130 may contain sterilizing gas. After being sterilized and sealed, the individual packages are packed into cases by robotic case packers 140. Alternatively, the packing may be done manually. The cases, perhaps corrugated boxes, may be formed by a case erector 160, which is located outside of the area 130. Alternatively, the cases may be erected manually inside or outside of the area, or the cases may be erected offsite, manually or automatically. Then, the cases are loaded onto pallets by a palletizer 150. Again, this operation could be done manually. From there, pallets 142 are moved into a degassing room 170 through an entrance 172. The pallets 142 rotate through the degassing room 170 via a conveyor system 174. After degassing is complete, the pallets exit the degassing room 170 through an exit 176. As an alternative to the conveyor system, the cases could be batchwise loaded into the degassing room where they remain stationary until degassing is complete. Then, all cases could be removed from the degassing room 170.

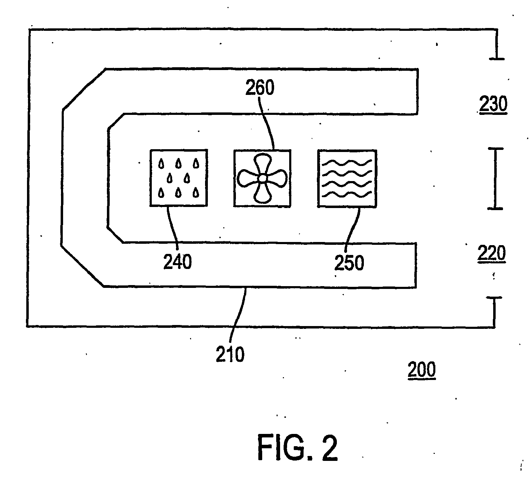

Fig. 2 shows the layout of a pretreatment area 200 through which articles may be sent prior to being packaged and sterilized. That is, articles may pass through the pretreatment area 200 before manipulation by the equipment shown in Fig. 1. The pretreatment area 200 may include a conveyor 210, which conveys articles from an entrance 220 to an exit 230. Humidity in the pretreatment area 200 is increased, perhaps with steam, through a humidifier 240 so that the relative humidity in the room is between about 40 and about 80 %. Heater 250 increases the temperature of the pretreatment area 200, perhaps to between 37.8°C and 60°C (about 100 and about 140 °F). If steam is used to supply moisture, the steam may also supply sufficient heat, eliminating the need for heater 250. If necessary, an air circulation system 260 can circulate the warm humidified air to increase convective heat transfer with the articles on the conveyor 210 and to more quickly introduce water to the articles. Squirrel cage fans may be used as the air circulation device 260. The conveyor 210 may be a slow moving conveyor, to keep the articles within the pretreatment area 200 for about 12 to about 72 hours. After pretreatment is complete, the articles are removed through the exit 230.

Moisture and heat serve important respective functions in the sterilization process. Moisture allows for the sterilizing gas to work effectively. Heat allows for sufficient sterilization to occur while the medical articles are exposed to peak sterilizing gas concentrations. With sufficient heating, the limiting factor in determining the degassing and quarantine time is the time required for the sterilizing gas to dissipate, not the time required for sterilization to finish. The length of time required for the degassing and quarantine processes can therefore be reduced. Moisture and heat can be supplied within the pretreatment area 200 as described above. Moisture and heat can also be supplied later in the process. If significant heat is not supplied later in the process, the medical articles should be heated to at least 26.7°C (80°F) or at least 37.8°C (100°F) within the pretreatment area 200.

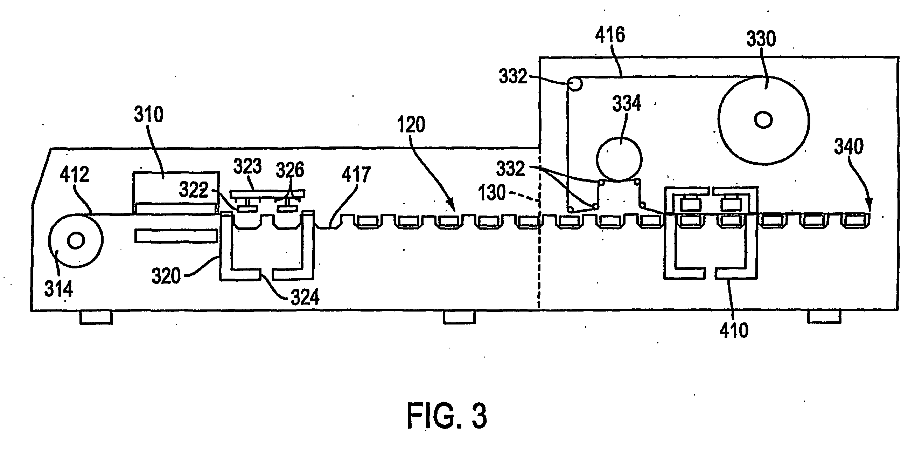

Fig. 3 is a schematic side view of the form, fill and seal device shown in Fig. 1. In Fig. 3, a bottom web 412 is provided from a roller 314. The bottom web 412 is then sent to a preheater 310. The preheater 310 softens the bottom web 412 so that it can be subsequently formed. Reference numeral 320 represents a forming machine. In this machine, plugs 322 form indentations 417 in the bottom web 412. These indentations 417 form cavities for the medical articles. An aperture 324 is provided within the forming machine 320. A vacuum is provided through the aperture 324 allowing the indentations 417 to be made in the bottom web 412. The vacuum from the bottom cooperates with pressure from the top. A plurality of knives 326 are provided on a plate 323 above the plugs. With this configuration, when the plugs 322 are at their lower most position to form the indentations 417, the knives 326 puncture the bottom web 412. Specifically, the knives 326 form small slits in the bottom web 412 to allow gas injection pins (not shown in Fig. 3) to fit therethrough. In one embodiment, these slits are formed on opposing sides of the housings 417.

In the embodiment shown in Fig. 3, the bottom web 412 is supplied from a roller 314. It is possible, however, that the indentations 417 would be preformed within the bottom web 412. In this case, the preheater 310 and forming machine 320 would not be necessary. If provided, the forming machine 320 simultaneously forms the indentations 417 and the slits. However, these steps could be done consecutively, with either the indentations or the slits being formed first. Further, it is not necessary for the slits to be formed by puncturing, as shown in Fig. 3. For example, the slits could be formed by positioning a roller closely adjacent to the bottom web 412. If the roller has objects protruding therefrom, these objects could make slits within the bottom web 412 as the projections contact the bottom web 412.

After the bottom web 412 is formed in the forming machine 320, medical articles from the pretreatment area are placed within the indentations 417 formed in the bottom web 412. This may be done at article loading area 120. Because this area of the form, fill and seal device is outside of the area 130, the articles may be placed in the indentations 417 by hand. After the indentations 417 have been filled, they move to the area 130.

Moisture and heat may be important to the sterilizing process. Moisture and heat may be supplied during pretreatment or during subsequent processing or during both pretreatment and subsequent processing. If moisture and heat are supplied only during pretreatment, it may be important that the medical articles be placed into the indentations 417 shortly after being removed from the pretreatment area 200. This allows for sufficient moisture and heat to remain with medical articles during sterilizing gas exposure. In one embodiment, the medical articles are left out of the pretreatment area for no more than 3 hours, and more particularly, for no more than 1 hour, before being loaded into the indentations 417, depending upon the rates of heat transfer and humidity dissipation from the medical articles at ambient temperature and relative humidity. This time can be extended if the medical articles are stored in a high relative humidity and/or high temperature atmosphere between the pretreatment area 200 and loading into the indentations 417.

A top web 416 is provided from a roll 330. The top web 416 is aligned with the bottom web 416 through a plurality of rollers 332. In a particular process, the top web 416 passes under a drum 334 of a flexographic printing press. With this embodiment, the top web 416 runs between a plate and the drum 334 so that product identification information can be printed on the top web 416. Within a sterilization-sealing station 410, described in detail later, the articles are sterilized with a sterilizing gas, and then, the top web 416 is sealed to the bottom web 412. Subsequently, at a cutting station 340, housings which are formed when the top and bottom webs 416, 412 are sealed, are separated into individual sterilized and packaged products.

Suitable sterilizing gases are at least compatible with the un-sterilized article and the processing parameters, such as temperature and pressure and, when present in sufficient quantity, can effectuate the sterilization of the article over a period of time. In one embodiment, the sterilizing gas is a mixture of a diluent gas and a sterilizing gas. These diluent gases reduce the flammability and inherent hazards associated with ethylene oxide. Diluent gases are those gases which are, at the least, compatible with both the sterilizing gas or gases and the article being sterilized. Examples of sterilizing gases include, but are not limited to, ethylene oxide, ozone, hydrogen peroxide vapor and plasma. Examples of diluent gases include, but are not limited to, nitrogen, carbon dioxide and fluorocarbons. When the sterilizing gas is supplied as a mixture of ethylene oxide and either nitrogen or carbon dioxide, the percent by volume of ethylene oxide present therein may generally be at least about 2%, and more particularly, from about 3% to about 25% and still more particularly, from about 5% to about 10% and still more particularly, from about 6% to about 8%.

Suitable gas mixing systems for mixing ethylene oxide and either nitrogen or carbon include batch and continuous systems. In either case, liquid ethylene oxide may be conveyed from a source via a conduit to a vaporizer or heat exchanger. The vaporizer or heat exchanger converts the liquid ethylene oxide into gaseous ethylene oxide.

The top and bottom webs, 416 and 412, may be formed from a variety of materials. Examples of materials suitable for forming the top web 416 include, but are not limited to, paper and paper polyolefin film laminates, plastic, polyolefin films, polyethylene films, high density polyethylene films and high density polyethylene film laminates, nylon 66, and polyolefin nonwoven fibers. Examples of materials suitable for forming the bottom web 412 include, but are not limited to, co-extruded ethylene-vinyl acetate, ethylene-vinyl acetate, ethylene-vinyl acetate laminates, particularly an ethylene-vinyl acetate/ionomer resin/ethylene-vinyl acetate laminate and polyethylene film. Ionomer resins are also know by the trademark SURLYN®.

In some instances the top and bottom web forming materials are suitable for the bonding or fusing together by a heating source, such as a heat bar or other conventional bonding or fusing source. Furthermore, in some instances the material forming the top web 416 and/or the bottom web 412 permits sufficient quantities of the sterilizing gas or gases introduced into the housing formed by the indentation 417 and the top web 416 to pass therethrough (degas). In this way, upon completion of the sterilization process, the sterilized articles may be removed from the housing without hazard or risk from residual levels of the sterilizing gas or gases. Upon closing the housing, such as by bonding or fusing portions of the top and bottom webs, 416 and 412, respectively, both the top web 416 and the bottom web 412 should be sufficiently impermeable to contaminating agents such as bacteria, viruses, dirt, fluids and the like.

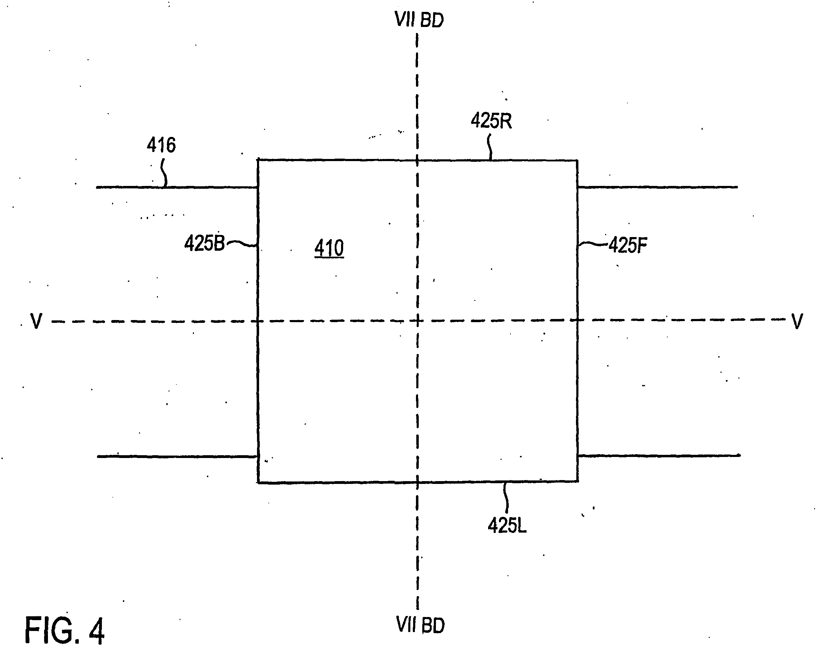

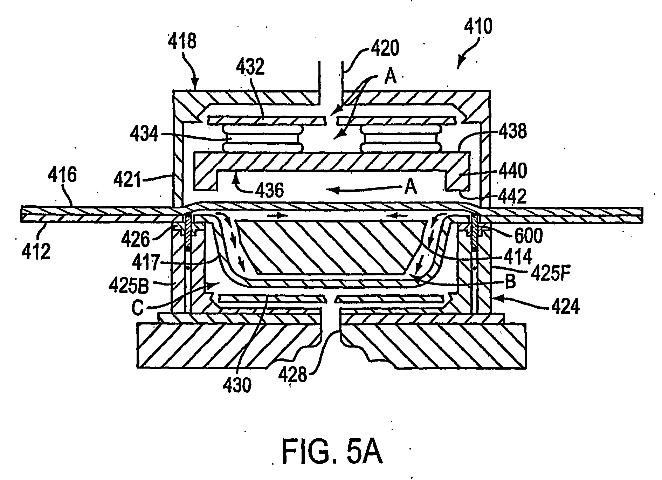

Fig. 4 is a top view of a sterilization-sealing station included in the form, fill and seal device shown in Figs. 1 and 3. Figs. 5A-5C are cross-sectional views of the sterilization-sealing station 410 taken through line V-V of Fig. 4. Figs. 5A-5C illustrate various stages of the sealing process. Fig. 5A is a side view of the sterilization-sealing station 410 illustrating the top and bottom webs 416, 412 clamped together, but not sealed. As shown in Fig. 5A, the sterilization-sealing station 410 includes a lid 418 having an upper gas port 420, and downwardly extending side walls 421. The lower most portion of the side walls 421 is provided with a continuous lip for engaging the upper surface of the top web 416. This can be configured in other ways to achieve same function.

A vertically adjustable seal die 424 includes upwardly extending side walls 425 having a continuous T-rubber seal 426 secured to the upper most portion the side walls 425. The seal die 424 further includes a lower gas port 428 and an apertured platform 430. The lid 418 and the seal die 424 are dimensioned such that a portion of the side wall 421 overlies a portion of the T-rubber seal 426.

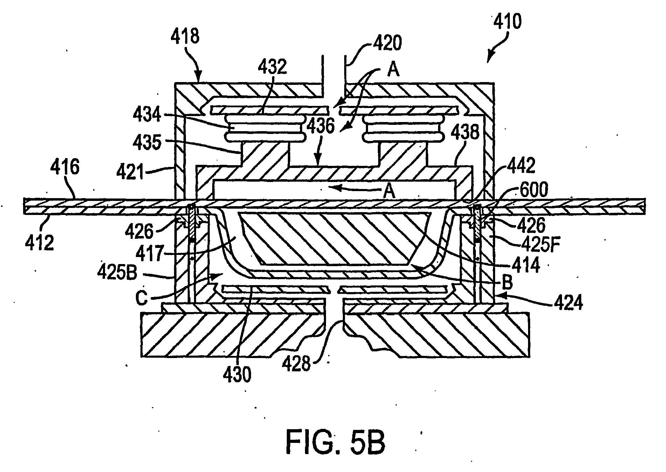

Secured to an apertured platform 432 within the lid 418 is a pair of cylinders 434, each including a piston 435 (Fig. 5B) which is adapted for vertical movement. The upper end of each cylinder 434 is secured to the platform 432. A heat sealer 436, having a horizontal surface 438 and downwardly extending side walls 440, is secured along the horizontal surface 438 to each of the pistons 435. The lower most portion of the side walls 440 is provided with a lip 442. The lip 442 of the heat sealer 436 and the seal die 424 are dimensioned such that a portion of the lip 442 overlies a portion of the T-rubber seal 426.

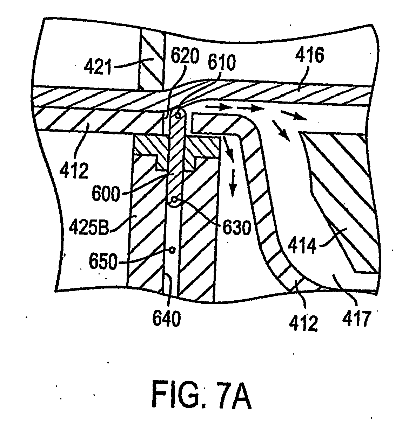

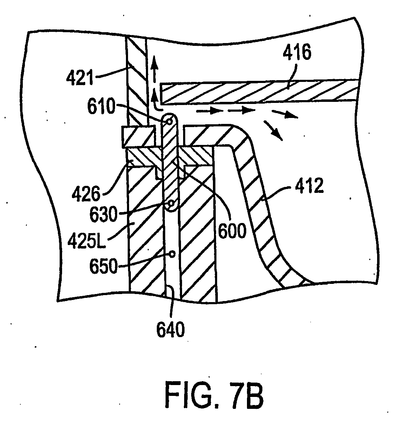

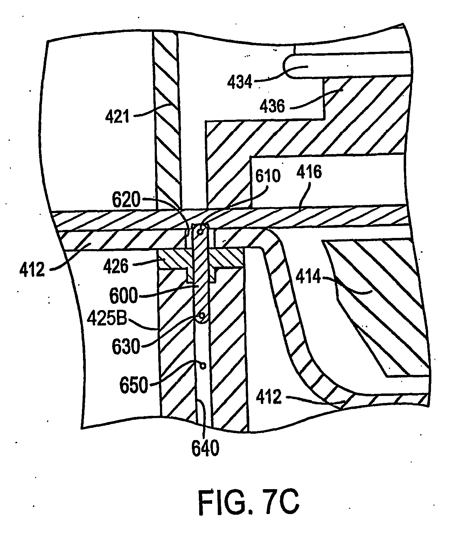

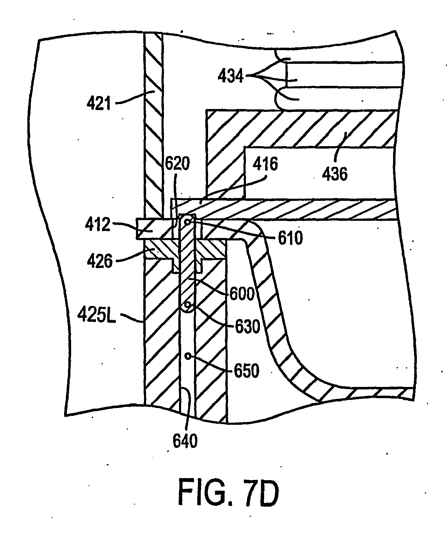

For the purpose of gas injection, pins 600 are provided within the side walls 425 of seal die 424. The side walls 425 located to the front, back, left and right of the sterilization-sealing station 410 are shown in various drawings. To differentiate front, back and left and right side walls, the letters "F," "B," "L," and "R" respectively are used after the reference numeral 425. Figs. 7A-7D are enlarged cross-sectional views showing a portion of the sterilization-sealing station 410 shown in Figs. 1, 3 and 4. Specifically, Fig. 7A is an enlarged cross-sectional view of the sterilization-sealing station 410 shown in Figs. 1, 3 and 4 taken through line V-V of Fig. 4, illustrating gas injection. Fig. 7B is an enlarged cross-sectional view showing a portion of the sterilization-sealing station 410 shown in Figs. 1, 3 and 4, taken through line VII BD-VII BD of Fig. 4, illustrating gas injection. Fig. 7C is an enlarged cross-sectional view showing a portion of the sterilization-sealing station shown in Figs. 1, 3 and 4, taken through line V-V of Fig. 4, illustrating a sealing operation. Fig. 7D is an enlarged cross-sectional view showing a portion of the sterilization-sealing station shown in Figs. 1, 3 and 4, taken through line VII BD-VII BD of Fig. 4, illustrating a sealing operation.

The pins 600 can be better seen in Figs. 7A-7D. Fig. 7A corresponds with Fig. 5A. Pins 600 have gas injection ports 610 at upper ends thereof, that fit through slits 620 in the bottom web 412. These slits 620 were made earlier, perhaps by knives 326 (see Fig. 3). Gas enters the pin 600 from a bottom port 630. A plurality of pins 600 are provided around the perimeter of the sterilization-sealing station 410. For each connection to a gas supply, a hole 640 extends through the side wall 425 of the die. In some instances, it may not be feasible to supply gas individually to each of the pins 600. Accordingly, to distribute gas from a single supply line to a plurality of pins 600, a conduit 650 may be formed within the side walls 425. The seal die 424 may have a square or rectangular perimeter. In this case, to form the conduit 650 within the side walls 425, the side walls 425 can simply be drilled from the corners to form four interconnecting holes through side walls 425.

Referring again to Fig. 5A, the evacuation process begins with positioning a formed bottom web 412, which supports a medical article 414, and a top web 416 within the sterilization-sealing station 410. At this point, the top and bottom webs, 416 and 412, respectively, are in loose contact.

In the next sequence of the evacuation process, the seal die 424 is elevated so as to contact and compress portions of the top and bottom webs, 416 and 412, respectively, against each other. Fig. 5A shows webs 416 and 412 in this compressed state, which is created by the respective forces exerted by the seal die 424 and the lid 418 against the bottom and top webs, 412 and 416, respectively. In this sterilization-sealing station configuration, the housing formed by the indentations 417 and the top web 416 is partially closed. The bottom and top webs, 412 and 416 are in compressive contact at the outer periphery of the housing because of the lid 418 and the seal die 424. However, the webs 412, 416 are not adhered together.

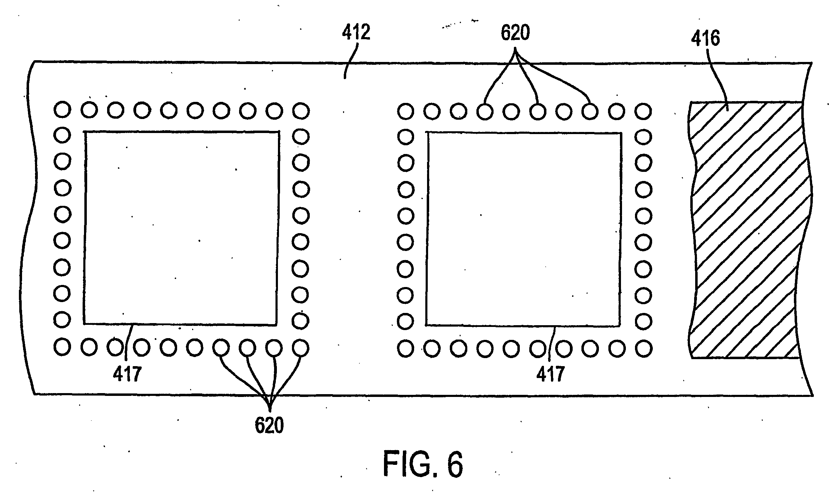

Fig. 6 is a top view showing the relative sizes of the bottom and top webs 412 and 416. Fig. 6 shows the slits 620 formed in the bottom web 412 for pin gas injection. Fig. 6 also shows the indentations 417 formed in the bottom web 412. As can be seen, the slits 620 extend around the indentations 417. Any configuration of the slits 620 is within the scope of the present invention. For example, the slits 620 may be positioned only along opposing sides of the indentation 417 or may be positioned only at the corners of the indentation 417. As can been seen in Fig. 6, the top web 416 is narrower than the bottom web 412. The reduced width of the top web 416 cannot be seen in Figs. 5A through 5C, 7A and 7C because these drawings are cross-sectional views taking through the length of the top and bottom webs 416, 412. That is, Figs. 5A through 5C, 7A and 7C are taken through the front and back side walls of the sterilization-sealing station. From this view, the webs 412, 416 would appear moving through the sterilization-sealing station from the right to the left of the drawings. Fig. 7B is an enlarged front cross-sectional view of the sterilization-sealing station 410 shown in Fig. 5A. Whereas Fig. 7A is a side cross-sectional view taken through the back side wall 425B, Fig. 7B is a front cross-sectional view taken through the left side wall 425L. In Fig. 7B, it is therefore possible to see the differing widths of the top and bottom webs 416, 412. The differing widths can also be seen in Fig. 7D. In operation, the top and bottom webs 416, 412 would appear to be moving into the plane of Figs. 7B and 7D. It should be noted that the sterilization-sealing station 410 may be symmetrical. In this case, a back view taken through the right sidewall would be symmetrical to Figs. 7B and 7D.

As mentioned above, the top web 416 has a width sufficient to cover the slits 620 in the bottom web 412 and cover the pin gas injection ports 610. Fig. 7B shows that the width of the top web 416 is less than the width of the sterilization-sealing station 410. In this manner, when the seal die 424 and the lid 418 come together, only the bottom web 412 is clamped at the left and right of the sterilization-sealing station 410, as shown in Fig. 7B. However, both the bottom and top webs 412, 416 are clamped at the front and back of the sterilization-sealing station 410, as shown in Fig. 5A.

As mentioned previously, the slits 620 may be formed only on the left and right sides of the bottom web 412. Referring to Fig. 6, the slits 620 positioned between the indentations 417 may be eliminated. Similarly, the gas injection pins 600 along the front and back walls 425F, 425B would be eliminated. With this alternative embodiment, the gas injection pins 600 and slits could not be seen in the views of Figs. 5A-5C, 7A and 7C. Figs. 7B and 7D show gas injection pins 600 provided within the left side wall 425L. The views of Figs. 7B and 7D would not change with the alternative embodiment. That is, on the left and right sides, gas injection pins 600 and slits 620 would remain.

Elevation of the seal die 424 to contact the lid 418 creates three chambers within the sterilization-sealing station 410. These three chambers are illustrated by the letters A, B and C. The chamber A is defined by the interior area of the lid 418 and the upper surface of the top web 416. The chamber B is defined by the indentation 417 and the overlaying top web 416. Chamber B is also referred to herein as the "housing." The chamber C is defined by the interior area of the seal die 424 and the lower surface of the bottom web 412. The chambers A, B and C are not in a completely sealed condition although chamber B is completely sealed at the end of the process. The pin gas slits 620 in the lower web 412 define some minimum connection between the chambers B and C. The narrowed width of the top web 416 provides gas communication between chambers A and B. Upper gas port 420 selectively communicates gas into and out of chamber A. Gas injection pins 600 selectively communicate gas into the chamber B. Lower gas port 428 selectively communicates gas into and out of chamber C. In selected embodiments, the sterilizing gas may be added only through the gas injection pins 600.

During the evacuation process, the pressure within the sterilization-sealing station 410 may be reduced to between about 30 and about 650 millibars. However, sterilizing gases containing ethylene oxide are believed to work better in the presence of moisture. Moisture may be added in the pretreatment area and/or during the sealing process. If moisture is only added in the pretreatment area, the evacuation process will remove some of this moisture. It is important, however, that a portion of the moisture remains with the medical article. The reduction in pressure during the evacuation process should be controlled so as to achieve this goal. In this case, the pressure after the evacuation process may be somewhat greater than the 30 to 650 millibars mentioned previously. In one embodiment, the reduced pressure should allow for the relative humidity within chamber B to be at least 40 % during the time period when ethylene oxide is injected.

After the evacuation process, gas is introduced. During this process, the sterilizing gas (alone or with diluent gas) described above, and perhaps steam, is introduced into the chamber B. Fig. 5A, and more particularly Figs. 7A and 7B, show gas (sterilizing gas and/or steam) being ejected from the gas injection pins 600. From there, at least some of the gas travels between the upper web 416 and the lower web 412 to enter the chamber B. A portion of the gas enters the chamber C, by traveling between the bottom web 412 and the T-rubber seal 426. Note that the front and back sidewalls 425F, 425B may be symmetrical, and the left and right side walls 425L, 425R may be symmetrical. Fig. 7A shows what happens at the front and back side walls 425F, 425B, where both the top and bottom webs 412, 416 are trapped between the seal die 424 and the lid 418. In this case, substantially no gas can escape from the gas injection pins 600 to the chamber A. On the other hand, Fig. 7B shows what happens at the left and right side walls 425, where only the bottom web 412 is trapped between the seal die 424 and the lid 418. As can be seen, gas can enter chamber A from the gas injection pins 600. The gas travels between the top web 416 and the side wall 421 of the lid 418.

If steam is supplied during the gas introduction sequence, the steam may be introduced before the sterilizing gas or simultaneously with the sterilizing gas. When the steam and the sterilization gas are introduced sequentially, steam may be first supplied to the chamber B through the gas injection ports 610. Then, the sterilization gas may be introduced, also through the gas injection ports 610. Steam is introduced into the chamber B until the pressure in the chamber B increases from about 40 to about 100 millibars. After the supply of steam is removed, the sterilization gas is introduced into the chamber B until the pressure in the chamber measures between about 300 and about 700 millibars. During this gas introduction process, it may seem necessary to partially eliminate the vacuum from the chamber A and C to prevent an uneven pressure and prevent chamber B from greatly expanding. The vacuum is released by the injection of steam and gas into chamber B. There is sufficient communication between chambers A, B and C such that the gas (steam) migrates to chambers A and C. It may not be necessary to release the vacuum through gas ports 420 and 428. The vacuum is removed through chamber communication so that the pressure in chambers A and C changes with the pressure in chamber B, to maintain substantially equal pressures in chambers A, B and C during the gas introduction process.

Moisture and heat sources (perhaps both through steam) are important to (1) enable the sterilizing gas to sterilize and (2) to ensure that there is sufficient sterilization when the medical articles are exposed to the peak concentrations of the sterilizing gas. Moisture and heat supplied during pretreatment, perhaps as steam, is one way to achieve these goals. In addition, or in the alternative to moisture and heat pretreatment, steam supplied at the sterilization-sealing station 410 of the form, fill and seal device can achieve these goals. In the conventional device, steam was added within the sterilization-sealing station 410 through a nozzle where condensation occurred. This steam may have supplied sufficient moisture to enable the sterilizing gas to sterilize. However, this steam may not have supplied sufficient heat to heat the medical articles. If there is no preheating, steam can be pulsed to a specified pressure through a steam valve.

Then, the steam valve can be closed and a vacuum applied through the gas ports 420 and 428 to evacuate a portion of the steam. This purge/pulse process is continued until the product 410 is heated to a desired temperature. In one embodiment, the chamber is evacuated to a pressure of 6.77 KPa (2 inches of mercury absolute). Then, steam is added to a pressure of 7.8 KPa (2.3 inches of mercury absolute). The pulse/purge process would be repeated 5 to 10 times or more to heat the product to greater than 37.8°C (100°F) or perhaps between 48.9°C and 54.4°C (about 120°F and about 130°F).

As an alternative to the pulse/purge process, high pressure steam may be injected. After evacuation, steam may be injected through the gas injection pins 600. The steam may be delivered to chamber B at a pressure of 414 to 689 KPa (60 to 100 psia), more specifically at a pressure between 483 and 621 KPa (70 and 90 psia). Because the top and bottom webs are not sealed at this point in the process, the steam passes from chamber B to chambers A and C until there is pressure equalization. Steam is allowed to enter the chamber B until the pressure equalizes to approximately the delivery pressure of the stream. The amount of heat supplied to the chamber by proceeding in this manner is based upon the sum of the volumes for chambers A, B and C. The amount of heat added is 372 to 1860 KJ/m$^3$ (10 to 50 Btu per cubic foot), and more specifically 1302 to 1674 KJ/m$^3$ (35 to 45 Btu per cubit foot).

Once the chamber is pressurized with steam, the pressure is maintained for a dwell period until the product is sufficiently heated. As mentioned before, the medical article should be heated to at least 26.7°C (80°F) or at least 37.8°C (100°F). To sufficiently heat the medical articles, a dwell time of 1 to 8 minutes, more particularly, 2 to 7 minutes, and even more particularly, 2.5 to 6.5 minutes may be used.

After the article is sufficiently heated, the chamber is again evacuated to a pressure between about 30 and 650 millibars. Then, the sterilizing gas can be introduced.

When the sterilization gas is introduced into the chamber B simultaneously with steam, the sterilization gas may either be pure or diluted. If the sterilization gas is 100% ethylene oxide, the percent by volume of ethylene oxide and other gases present within the chamber B may be within the following ranges: ethylene oxide-between about 2% and about 50%; steam-between about 2% and about 20% and air-between about 0% and about 78%. When the sterilization gas introduced into the chamber B is a combination of ethylene oxide and a diluent gas, the percent by volume of these gases and other gases present the chamber B may be within the following ranges: ethylene oxide-between about 2% and about 25%; diluent gas between about 25% and about 96%; steam-between about 2% and about 20%; and air-between about 0% and about 30%. When the diluent gas is nitrogen, the percent by volume thereof in the chamber B may be between about 25% and about 96%, and particularly between about 60% and about 90%, and more particularly between about 65% and about 85% and still more particularly between about 70% and about 80%. When the diluent gas is carbon dioxide, the percent by volume thereof in the chamber B may be between about 25% and about 96% and particularly between about 60% and about 90% and more particularly between about 75% and about 85% and still more particularly between about 70% and about 80%.

It is further possible to combine the sequential and simultaneous steam and sterilization gas introduction processes. In this manner, steam may be introduced first. Then, both steam and the sterilization gas may be introduced.

With the conventional chamber sterilization process, there were numerous variables that controlled the amount of sterilizing gas remaining in the housing after the sterilization step. With the form, fill and seal device, the present invention can accurately determine the amount and concentration of residual sterilizing gas. The ethylene oxide concentration is calculated on the basis of the difference in total pressure resulting from the addition of ethylene oxide plus carrier or diluent gas, and on the basis of the temperature of the sterilization-sealing station 410. The difference in total pressure due to the addition of ethylene oxide and diluent gas can be expressed as follows:

$$P = P_{EO} + P_{DG} = \left( \left( \frac{n}{v} \right)_{EO} + \left( \frac{n}{v} \right)_{DG} \right) RT$$

where $P_{EO}$ and $P_{DG}$ are the partial pressures resulting from the addition of ethylene oxide and diluent gas, respectively, n and v are the number of moles and the volume of gas added, R is the gas constant and T is the absolute temperature of the gas added.

Rearranging the Ideal Gas Law allows for the calculation of ethylene oxide concentration, C in mg/l regardless of the diluent, using the following equation:

$$C = \frac{K \times P}{R \times T}$$

where K is a constant for a given particular sterilizing gas. For ethylene oxide, K is defined as follows:

$$K = \frac{4.4 \times 10^4 (M)(E)}{(M)(E) + 44(100 - E)}$$

where M is the molecular weight of the diluent gas and E is the weight percent of ethylene oxide in the diluent/sterilizing gas combination. In the denominator, it should be noted that 44 is the molecular weight of the ethylene oxide.

The concentration of residual sterilizing gas, such as ethylene oxide, within chamber B (the housing) should be greater that 50 milligrams per liter, perhaps greater than 100 milligrams per liter or perhaps within the range of 200 to 400 milligrams per liter. A concentration meeting these requirements may be obtained by injecting 8.6 wt. % ethylene oxide and 91.4 wt. % HCFC-124 such that after gas injection, the pressure is increased by 69.4 KPa (20.5 inches of mercury absolute). If the temperature during the injection process was 55°C, then:

$$P = 20.5 \text{ inHg} = 0.69 \text{ atm}$$

$$T = 55°C = 328°K$$

$$R = 0.08205 \frac{atm \quad l}{gm \quad mole \quad °K}$$

$$K = \frac{4.4 \times 10^4 \, ME}{ME + 44(100 - E)} = \frac{(4.4 \times 10^4)(124)(8.6\%)}{(124)(8.6\%) + 44(100 - 8.6\%)} = 9.22 \times 10^3 \, mg / gm \quad mole$$

$$C = \frac{K \times P}{R \times T} = \frac{(9.22 \times 10^3)(0.69)}{(0.08205)(328)} = 236.4 mg / l$$

After the gas introduction process, the top and bottom web 416, 412 are adhered together in a heat sealing sequence. Fig. 5B illustrates the sealing sequence. In this sequence, the supply of gases is stopped, and the gases previously introduced into the chamber B are captured therein. A heat sealer 436 is moved downward toward the seal die 424, by the extension of the pistons 435 such that a lip 442 of the heat sealer 436 contacts the upper surface of the top web 416.

Figs. 7C and 7D are enlarged partial cross-sectional views of the device shown in Fig. 5B. Fig. 7C is a side cross-sectional view taking through a back side wall 425B. Fig. 7D is a front cross-sectional view taking through a left side wall 425L. As can be seen, in both drawings, both the top web 416 and the bottom web 412 are pinched between the heat sealer 436 and the seal die 424. In Fig. 7D, however, only the bottom web 412 is clamped between the side wall 421 of the lid 418 and the T-rubber seal 426.

Upon the application of sufficient pressure and heat by the heat sealer 436 to the top web 416 and after the passage of sufficient time, the top and bottom webs 416 and 412 become secured together, such as by bonding or fusing. This action closes chamber B. Ventilation of the chambers A and C via ports 420 and 428, respectively, begins once the seal is formed so that residual sterilization gas may be removed from chambers A and C after chamber B is closed within the sterilization-sealing station 410.

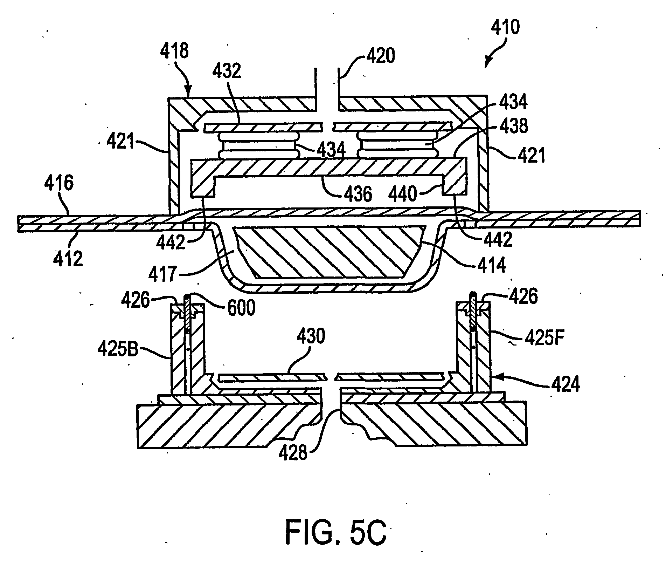

Referring now to Fig. 5C, the heat sealer 436 has been raised by retracting the pistons 435 (not shown) such that lips 442 are spaced away from the top web 416. The seal die 424 has been retracted such that the T-rubber seals 426 are spaced away from the bottom web 412. The closed housing (chamber B) is now advanced by a conveyer system to exit the sterilization-sealing station 410. Generally, simultaneously with the advancement of the closed housing, an indentation 417 supporting a non-sterilized medical article enters the sterilization-sealing station 410 and the sealing sequence is repeated. Thus, before the lid 418 and the seal die 424 clamp the webs 416 and 412 together as shown in Fig. 5A, the lid 418 and the seal die 424 are separated, as shown in Fig. 5C to allow entry of the indentation 417 and the top web 416.

After being sealed, the individual packages are packed in the cases by robotic case packers 140 (see Fig. 1). The cases are then pelletized by palletizer 150 for transportation into a degassing room 170. The individual packages could also be dropped into bins and manually case packed after degassing. The temperature within the area 130 and particularly the degassing room 170 may be maintained from 21.1°C to 71.1°C (about 70°F. to about 160°F). and particularly from 32.2°C to 65.6°C (about 90°F. to about 150°F). and more particularly from 48.9°C to 60°C (about 120°F. to about 140°F). The temperature within the area 130 and the degassing room 170 may be maintained above 71.1°C (160°F). provided that the article being sterilized and the materials forming the top and bottom webs 416, 412 are compatible with the elevated temperature. The pelletized housings remain in the degassing room 170 for a sufficient time to effect degassing. This period of time is generally at least about 4 hours and particularly from about 4 hours to about 48 hours.

The length of the degassing process can be determined based on the amount of residual sterilizing gas and the rate of diffusion of same from the housing (chamber B) to the surrounding environment.

It may be undesirable to directly vent the ethylene oxide removed in the degassing room 170. Accordingly, an ethylene oxide eliminator system (not shown) may be used. The ethylene oxide eliminator system functions to control ethylene oxide emission into the atmosphere. Such systems generally use catalytic oxidation technology to convert ethylene oxide into carbon dioxide and water vapor. One such ethylene oxide eliminator system, the ETO-Abator™, is available from the Donaldson Company, Inc. of Minneapolis, Minnesota.

While the invention has been described in detail with respect to specific embodiments thereof, it will be appreciated that those skilled in the art, upon attaining an understanding of the forgoing may readily conceive of alterations to, variations of and equivalents to these embodiments. Accordingly, the scope of the present invention should be assessed as that of the appended claims.

## Claims

1.   A medical article sterilization device comprising :

a pretreatment area (200) for medical articles, the pretreatment area having a heat source (250) to heat the medical articles;
a device (320) to form a housing in a first web;
an article loading station (120) where a medical article (414) heated in the pretreatment area is loaded into the housing in the first web;
an alignment device (332) to align a second web (416) with the first web; and
a sterilization-sealing station (410) where the first web and the second web, with the medical article loaded into the housing, are sterilized and then the first and second webs are sealed together.

2.   A medical article sterilization device according to claim 1, wherein the sterilization-sealing station further comprises gas injection pins (600) to inject gas into the housing, between the first and second webs.

3.   A medical article sterilization device according to claim 2, wherein the sterilization-sealing station further comprises a steam source to inject steam into the housing, between the first and the second webs.

4.   A medical article sterilization device according to claim 1, wherein the sterilization-sealing station further comprises a steam source to supply steam to the housing.

5.   A medical article sterilization device according to claim 1, wherein:

substantially no moisture is supplied to the medical articles at the sterilization-sealing station, and
the pretreatment area has a steam source to supply moisture to the medical articles.

6.   A medical article sterilization device according to claim 5, wherein:

the sterilization-sealing station comprises a vacuum source and a controller,
the vacuum source evacuates the housing,
evacuating the housing removes moisture from the medical articles, and
the controller maintains the pressure in the housing so as to allow some moisture to remain with the medical articles.

**7.** A medical article sterilization device according to claim 6, wherein the controller maintains the pressure in the housing so as to allow the relative humidity in the housing to be at least 40% during sterilization gas exposure.

**8.** A method of sterilizing a medical article comprising:

preheating a medical article in a pretreatment area;
forming a housing in a first web;
loading the medical article heated in the pretreatment area into the housing formed in the first web;
aligning a second web with the first web;
sterilizing the medical article located in the housing and between the first and second webs;
after sterilizing the medical article, sealing the first web to the second web, with the medical article located in the housing and between the first and second webs.

**9.** A method of sterilizing a medical article according to claim 8, further comprising injecting gas into the housing between the first and second webs, through gas injection pins.

**10.** A method of sterilizing a medical article according to claim 9, wherein steam is injected into the housing, between the first and second webs, through the gas injection pins.

**11.** A method of sterilizing a medical article according to claim 8, wherein steam is injected into the housing, between the first and second web.

**12.** A method of sterilizing a medical article according to claim 8, wherein:

sterilization and sealing are conducted at a sterilization-sealing station,
substantially no moisture is supplied to the medical article at the sterilization-sealing station, and
steam is supplied to the medical article at the pretreatment area.

**13.** A method of sterilizing a medical article according to claim 12, further comprising the steps of:

evacuating the housing at the sterilization-sealing station so as to remove moisture from the medical article, and
maintaining the pressure in the housing so as to allow some moisture to remain with the medical article.

**14.** A method of sterilizing a medical article according to claim 13, wherein the pressure in the housing is maintained so as to allow the relative humidity in the housing to be at least 40% during sterilization gas exposure.


**Patentansprüche**

**1.** Ein Sterilisationsgerät für medizinische Artikel, umfassend:

Einen Vorbehandlungsbereich (200) für medizinische Artikel, wobei der Vorbehandlungsbereich eine Wärmequelle (250) aufweist, um die medizinischen Artikel zu erhitzen;
ein Gerät (320), um ein Gehäuse in einer ersten Bahn zu bilden;
eine Ladungsstation (120) für Artikel, in der ein medizinischer Artikel (414), der in dem Vorbehandlungsbereich erhitzt wurde, in das Gehäuse in der ersten Bahn geladen wird;
ein Ausrichtungsgerät (332), um eine zweite Bahn (416) nach der ersten Bahn auszurichten; und
eine Sterilisations-Dichtungsstation (410), in der die erste Bahn und die zweite Bahn sterilisiert und dann die erste und die zweite Bahn miteinander abgedichtet werden, wobei der medizinische Artikel in dem Gehäuse geladen ist.

**2.** Ein Sterilisationsgerät für medizinische Artikel nach Anspruch 1, wobei die Sterilisations-Dichtungsstation zusätzlich Gaseinblasungsstifte (600) umfasst, um Gas in das Gehäuse einzublasen, zwischen der ersten und zweiten Bahn.

3. Ein Sterilisationsgerät für medizinische Artikel nach Anspruch 2, wobei die Sterilisations-Dichtungsstation zusätzlich eine Dampfquelle umfasst, um Dampf in das Gehäuse einzublasen, zwischen der ersten und der zweiten Bahn.

4. Ein Sterilisationsgerät für medizinische Artikel nach Anspruch 1, wobei die Sterilisations-Dichtungsstation zusätzlich eine Dampfquelle umfasst, um Dampf in das Gehäuse zuzuführen.

5. Ein Sterilisationsgerät für medizinische Artikel nach Anspruch 1, wobei:

   den medizinischen Artikeln in der Sterilisations-Dichtungsstation im Wesentlichen keine Feuchtigkeit zugeführt wird, und
   der Vorbehandlungsbereich eine Dampfquelle aufweist, um den medizinischen Artikeln Feuchtigkeit zuzuführen.

6. Ein Sterilisationsgerät für medizinische Artikel nach Anspruch 5, wobei:

   die Sterilisations-Dichtungsstation eine Vakuumquelle und eine Kontrolleinheit umfasst,
   die Vakuumquelle das Gehäuse absaugt,
   das Absaugen des Gehäuses Feuchtigkeit von den medizinischen Artikeln entfernt, und
   die Kontrolleinheit den Druck in dem Gehäuse aufrechterhält, um zu ermöglichen, dass etwas Feuchtigkeit bei den medizinischen Artikeln zurückbleibt.

7. Ein Sterilisationsgerät für medizinische Artikel nach Anspruch 6, wobei die Kontrolleinheit den Druck in dem Gehäuse aufrechterhält, so dass ermöglicht wird, dass die relative Humidität in dem Gehäuse mindestens 40% beträgt während der Einwirkung des Sterilisationsgases.

8. Ein Verfahren zum Sterilisieren eines medizinischen Artikels, umfassend:

   Vorerhitzen eines medizinischen Artikels in einem Vorbehandlungsbereich;
   Bilden eines Gehäuses in einer ersten Bahn;
   Laden des medizinischen Artikels, der in dem Vorbehandlungsbereich erhitzt wurde, in das in der ersten Bahn gebildete Gehäuse;
   Ausrichten einer zweiten Bahn nach der ersten Bahn;
   Sterilisieren des medizinischen Artikels, der sich in dem Gehäuse zwischen der ersten und der zweiten Bahn befindet;
   Abdichten der ersten Bahn mit der zweiten Bahn, wobei der medizinische Artikel sich in dem Gehäuse und zwischen der ersten und der zweiten Bahn befindet, nach dem Sterilisieren des medizinischen Artikels.

9. Ein Verfahren zum Sterilisieren eines medizinischen Artikels nach Anspruch 8, welches zusätzlich das Einblasen von Gas durch Gaseinblasungsstifte in das Gehäuse zwischen der ersten und der zweiten Bahn umfasst.

10. Ein Verfahren zum Sterilisieren eines medizinischen Artikels nach Anspruch 9, wobei Dampf durch die Gaseinblasungsstifte in das Gehäuse zwischen der ersten und der zweiten Bahn eingeblasen wird.

11. Ein Verfahren zum Sterilisieren eines medizinischen Artikels nach Anspruch 8, wobei Dampf in das Gehäuse zwischen der ersten und der zweiten Bahn eingeblasen wird.

12. Ein Verfahren zum Sterilisieren eines medizinischen Artikels nach Anspruch 8, wobei
    das Sterilisieren und das Abdichten in einer Sterilisations-Dichtungsstation ausgeführt werden,
    dem medizinischen Artikel in der Sterilisations-Dichtungsstation im Wesentlichen keine Feuchtigkeit zugeführt wird, und
    dem medizinischen Artikel in dem Vorbehandlungsbereich Dampf zugeführt wird.

13. Ein Verfahren zum Sterilisieren eines medizinischen Artikels nach Anspruch 12, zusätzlich folgende Schritte umfassend:

    Absaugen des Gehäuses in der Sterilisations-Dichtungsstation, um Feuchtigkeit von dem medizinischen Artikel zu entfernen, und

Aufrechterhalten des Drucks in dem Gehäuse, um zu ermöglichen, dass etwas Feuchtigkeit bei dem medizinischen Artikel zurückbleibt.

**14.** Ein Verfahren zum Sterilisieren eines medizinischen Artikels nach Anspruch 13, wobei der Druck in dem Gehäuse aufrechterhalten wird, so dass ermöglicht wird, dass die relative Humidität in dem Gehäuse mindestens 40% beträgt während der Einwirkung des Sterilisationsgases.

**Revendications**

**1.** Dispositif de stérilisation pour article médical, comprenant :

une zone de prétraitement (200) pour articles médicaux, la zone de prétraitement ayant une source de chaleur (250) pour chauffer les articles médicaux ;
un dispositif (320) pour former un logement dans un premier voile ;
une station (120) de chargement d'articles, où un article médical (414), chauffé dans la zone de prétraitement, est chargé dans le logement formé dans le premier voile ;
un dispositif d'alignement (332) pour aligner un second voile (416) avec le premier voile ; et
une station (410) de stérilisation-soudage, dans laquelle le premier voile et le second voile, avec l'article médical chargé dans le logement, sont stérilisés, puis les premier et second voiles sont soudés ensemble.

**2.** Dispositif de stérilisation pour article médical selon la revendication 1, dans lequel la station de stérilisation-soudage comprend, en outre, des aiguilles (600) d'injection de gaz pour injecter du gaz dans le logement, entre les premier et second voiles.

**3.** Dispositif de stérilisation pour article médical selon la revendication 2, dans lequel la station de stérilisation-soudage comprend, en outre, une source de vapeur pour injecter de la vapeur dans le logement, entre les premier et second voiles.

**4.** Dispositif de stérilisation pour article médical selon la revendication 1, dans laquelle la station de stérilisation-soudage comprend, en outre, une source de vapeur pour fournir de la vapeur au logement.

**5.** Dispositif de stérilisation pour article médical selon la revendication 1, dans lequel :

sensiblement aucune humidité n'est fournie aux articles médicaux au niveau de la station de stérilisation-soudage, et
la zone de prétraitement comporte une source de vapeur pour fournir de l'humidité aux articles médicaux.

**6.** Dispositif de stérilisation pour article médical selon la revendication 5, dans lequel :

la station de stérilisation-soudage comprend une source de vide et un organe de commande,
la source de vide pompe le logement,
le pompage du logement élimine de l'humidité depuis les articles médicaux, et
l'organe de commande maintient la pression dans le logement de manière à permettre qu'un peu d'humidité reste dans les articles médicaux.

**7.** Dispositif de stérilisation pour article médical selon la revendication 6, dans lequel l'organe de commande maintient la pression dans le logement de manière à permettre à l'humidité relative, dans le logement, d'être au moins de 40% durant l'exposition au gaz de stérilisation.

**8.** Procédé de stérilisation pour article médical comprenant :

le préchauffage d'un article médical dans une zone de prétraitement ;
la formation d'un logement dans un premier voile ;
le chargement de l'article médical, chauffé dans la zone de prétraitement, dans le logement formé dans le premier voile ;
l'alignement d'un second voile avec le premier voile ;
la stérilisation de l'article médical situé dans le logement et entre les premier et second voiles ; et

après stérilisation de l'article médical, le soudage du premier voile au second voile, avec l'article médical situé dans le logement et entre les premier et second voiles.

**9.** Procédé de stérilisation d'un article médical selon la revendication 8, comprenant, en outre, l'injection de gaz dans le logement, entre les premier et second voiles, via des aiguilles d'injection de gaz.

**10.** Procédé de stérilisation pour article médical selon la revendication 9, dans lequel de la vapeur est injectée dans le logement, entre les premier et second voiles, via les aiguilles d'injection de gaz.

**11.** Procédé de stérilisation pour article médical selon la revendication 8, dans lequel de la vapeur est injectée dans le logement, entre les premier et second voiles.

**12.** Procédé de stérilisation pour article médical selon la revendication 8, dans lequel :

la stérilisation et le soudage sont réalisés au niveau d'une station de stérilisation-soudage, sensiblement aucune humidité n'est fournie à l'article médical au niveau de la station de stérilisation-soudage, et de la vapeur est fournie à l'article médical au niveau de la zone de prétraitement.

**13.** Procédé de stérilisation pour article médical selon la revendication 12, comprenant, en outre, les étapes suivantes :

le pompage du logement au niveau de la station de stérilisation-soudage de manière à éliminer de l'humidité depuis l'article médical, et
le maintien de la pression dans le logement de manière à permettre qu'un peu d'humidité reste dans l'article médical.

**14.** Dispositif de stérilisation pour article médical selon la revendication 13, dans lequel la pression dans le logement est maintenue de manière à permettre à l'humidité relative, dans le logement, d'être au moins de 40% durant l'exposition au gaz de stérilisation.

## FIG. 1

**FIG. 2**

FIG. 3

EP 1 347 786 B1

EP 1 347 786 B1

VII BD

425R

416

425B 410 425F

V — — — — — — — — — — — — — — — — — V

425L

FIG. 4

VII BD

FIG. 5A

FIG. 5B

EP 1 347 786 B1

FIG. 5C

FIG. 6

FIG. 7A

FIG. 7B

FIG. 7C

EP 1 347 786 B1

EP 1 347 786 B1

FIG. 7D